(19) Europäisches Patentamt
European Patent Office
Office européen des brevets

(11) Veröffentlichungsnummer: **0 022 512**
**A1**

(12) **EUROPÄISCHE PATENTANMELDUNG**

(21) Anmeldenummer: **80103722.7**

(22) Anmeldetag: **01.07.80**

(51) Int. Cl.³: **C 07 F 9/40**, C 07 F 9/32, C 07 F 9/44, C 07 F 9/36, C 07 F 9/65, C 09 B 29/32, C 09 B 31/10, C 09 B 35/035, G 03 C 1/52, C 07 D 231/22, C 07 D 231/34

(30) Priorität: **12.07.79 DE 2928136**

(43) Veröffentlichungstag der Anmeldung: **21.01.81 Patentblatt 81/3**

(84) Benannte Vertragsstaaten: **CH DE FR GB LI**

(71) Anmelder: **BAYER AG, Zentralbereich Patente, Marken und Lizenzen, D-5090 Leverkusen 1, Bayerwerk (DE)**

(72) Erfinder: **Bornatsch, Wolfgang, Dr., Baldurstrasse 21, D-5000 Köln (DE)**
Erfinder: **Leverenz, Klaus, Dr., Heymannstrasse 44, D-5090 Leverkusen 1 (DE)**

(54) **Phosphorhaltige Azoverbindungen sowie deren Herstellung und Verwendung als Schädlingsbekämpfungsmittel, lichtempfindliche Materialien und als Ausgangsstoffe zur Synthese von Pyrazolonen.**

(57) Azophono- bzw. -phosphinobernsteinsäureester der Formel

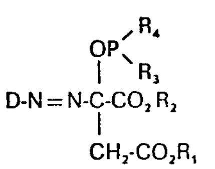

worin
D für den Rest einer Diazokomponente
$R_1$ für gegebenenfalls substituiertes Alkyl, Alkenyl, Aryl, Cycloalkyl, Aralkyl oder Heteroalkyl
$R_2$ für $R_1$
$R_3$ für $OR_1$ oder 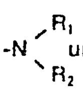 und
$R_4$ für $R_1$ oder $R_3$ stehen,
sind vielseitig verwendbare Verbindungen, die wegen ihrer Lichtempfindlichkeit im Diazotypieverfahren sowie wegen ihrer insektiziden und akariziden Wirkung als Schädlingsbekämpfungsmittel eingesetzt werden können. Beim Behandeln mit Basen entstehen Pyrazolone der Formeln

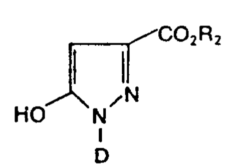

und

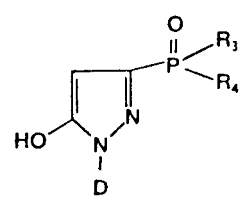

die wiederum Kupplungskomponenten zur Synthese von Azofarbstoffen darstellen.

EP 0 022 512 A1

BAYER AKTIENGESELLSCHAFT
Zentralbereich
Patente, Marken und Lizenzen

5090 Leverkusen, Bayerwerk
K-by

# Phosphorhaltige Azoverbindungen sowie deren Herstellung und Verwendung als Schädlingsbekämpfungsmittel, lichtempfindliche Materialien und als Ausgangsstoffe zur Synthese von Pyrazolonen

Gegenstand der Erfindung sind Azophosphono- bzw. -phosphinobernsteinsäureester der Formel

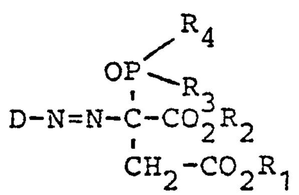

(I)

worin

D für den Rest einer Diazokomponente

$R_1$ für gegebenenfalls substituiertes Alkyl, Alkenyl, Aryl, Cycloalkyl, Aralkyl oder Heteroalkyl

$R_2$ für $R_1$

$R_3$ für $OR_1$ oder 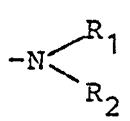 und

$R_4$ für $R_1$ oder $R_3$ stehen.

Geeignete Rest D sind solche der Benzol-, Naphthalin-, Anthrachinon- sowie der Pyrazol-, Imidazol-, Thiazol-, Indazol-, Thiadiazol-, Thiophen- und Benzthiazolreihe, wobei die Benzolreste bevorzugt sind.

Diese Reste können in der Azochemie übliche Substituenten, wie Alkyl, Alkoxy, Phenyl, Phenoxy, Alkylcarbonyl, Halogen, Cyan, Azophenyl, Nitro, Alkylsulfonyl, Arylsulfonyl, Alkoxycarbonyl, Alyklcarbonylamino, Arylcarbonylamino, Carboxyl, Hydroxyl und $SO_3H$, tragen.

Geeignete im beliebigen Zusammenhang genannte Alkylreste weisen1-5 C-Atome auf. Auch diese Reste können weitersubstituiert sein, z.B. durch OH, CN, Alkoxy, COOH, $HSO_3$-O oder Halogen.

Geeignete Cycloalkylreste sind z.B. Cyclohexylreste. Geeignete Arylreste sind Phenylreste, die z.B. durch Alkyl, Alkoxy, Halogen oder $SO_3H$ substituiert sein können.

Geeignete Aralkylreste sind Phenyl-$C_1$-$C_4$-Alkylreste die im Phenylkern durch Alkyl, Alkoxy oder Halogen substituiert sein können.

Geeignete Hetalkylreste weisen ebenfalls 1-4 C-Atome in der Alkylenkette auf. Beispielhaft seien genannt

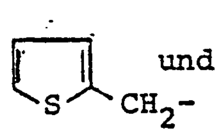 und 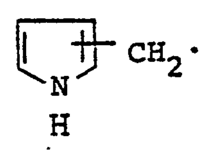

Geeignete Alkenylreste sind Vinyl, Allyl, Butenyl und Pentenyl.

Unter "Halogen" wird vor allem F, Br und insbesondere Cl verstanden.

Bevorzugte neue Azoverbindungen sind solche der Formel (I), worin

D für 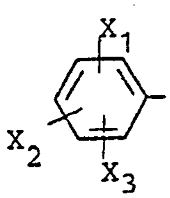

$R_1$ und $R_2$ für $C_1$-$C_4$-Alkyl, vorzugsweise $C_1$-$C_2$-Alkyl,

$R_3$ $OR_1$ und

$R_4$ für $R_1$ oder - vorzugsweise - $OR_1$ stehen,

wobei $X_1$ Wasserstoff, Cl, Br, $CH_3$, $OCH_3$ oder $CF_3$

$X_2$ CN, $NO_2$ Phenylazo, $C_1$-$C_2$-Alkylcarbonylamino oder $CF_3$

$X_3$ CHO, $CO_2$-$C_1$-$C_2$-Alkyl, Phenylsulfonyl, $SO_3H$, $C_1$-$C_2$-Alkylcarbonyl, $C_1$-$C_2$-Alkylsulfonyl oder $X_2$ bedeuten.

Geeignete Verbindungen der Formel (I) sind auch solche, in denen D für den Rest einer bifunktionellen Komponente, d.h. einer Tetrazokomponente, steht; sie entsprechen der Formel

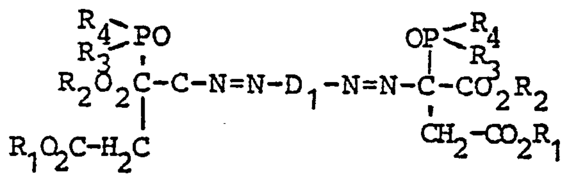 (II)

worin

$R_1$-$R_4$ die obengenannte allgemeinen und speziellen Bedeutungen haben.

Man erhält die neuen Azoverbindungen beispielsweise dadurch, daß man diazotierte Amine der Formel

$$D\text{-}NH_2 \qquad (III)$$

auf Phosphonobernsteinsäureester der Formel

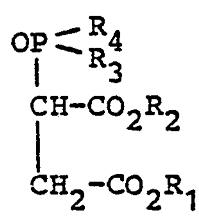 (IV)

in Gegenwart säurebindender Mittel kuppelt.

Die Kupplung erfolgt in wäßrigem oder wäßrig-organischem Medium bei Temperaturen von-10°C bis 30°C und einem pH von 5-10.

Geeignete Amine der Formel (III) sind übliche aus der Azochemie bekannte Verbindungen, wie z.B. Anilin, o-, m-, p-Toluidin, o-, m-, p-Nitranilin, 2,4-Dichloranilin, Kresidin, 1,4- und 1,3-Phenylendiamin, Dichlorbenzidin, Dianisidin, Sulfanilsäure, Dichlor-sulfanilsäure,

und die Verbindung 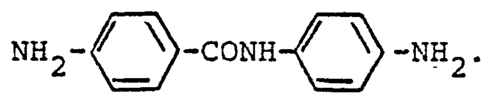.

Die Bernsteinsäureester der Formel (IV) sind bekannt bzw. nach bekannten Methoden (vgl. US-PS 3 801 682 u. J.A.C.S. 98, 1204 (1976)) leicht erhältlich. In der nachstehenden Tabelle sind einige Verbindungen beispielhaft angegeben.

Verbindungen der Formel (IV):

| $R_1$ | $R_2$ | $R_3$ | $R_4$ |
|---|---|---|---|
| Me | Me | OMe | OMe |
| Me | Me | OEt | OEt |
| Me | Me | O-Prop. | O-Prop. |
| Me | Me | O-Allyl | O-Allyl |
| Me | Me | O-Vinyl | O-Vinyl |
| Me | Me | O-Isopropyliden | O-Isopropyliden |
| Me | Me | O-Cyclohexyl | O-Cyclohexyl |
| Et | Et | O-Et. | O-Et |
| nBu | nBu | O-n-Bu | O-n-Bu |
| Me | Me | OMe | 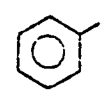 |
| Me | Me | 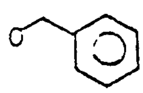 | Me |
| Oct. | Oct. | O-Oct. | O-Oct. |
| Me | Me | O-nBu | -OMe |
| Me | Me | $NMe_2$ | $NMe_2$ |

| $R_1$ | $R_2$ | $R_3$ | $R_4$ |
|---|---|---|---|
| Et | Et | $NEt_2$ | $NEt_2$ |
| nBu | nBu | O-Benzyl | O-Benzyl |
| Oct. | Oct. | OMe | 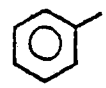 |
| Me | Me | n-Oct. | n-Oct. |
| Et | Et | dec | dec |

Als säurebindende Mittel seien beispielhaft genannt: Erdalkali-, Alkali- oder Ammoniumhydroxide, Erdalkali-, Alkali- oder Ammoniumcarbonate bzw. -hydrobencarbonate, Calciumoxid, Magnesiumoxid, Zinkoxid, Alkaliphosphate, Alkaliacetate sowie organische Amine wie: Pyridin, Chinolin, Triethylamin, Triethanolamin, Ethanolamin.

Die neuen Azoverbindungen sind - sofern sie keine hydrophilierenden Gruppen, wie $SO_3H$, OH, SH, COOH, enthalten - in Wasser unlösliche, ölige oder feste Substanzen, die in üblicher Weise durch Extrahieren, Abdekantieren oder Filtrieren aus dem Reaktionsgemisch isoliert und gegebenenfalls durch Umkristallisieren gereinigt werden.

Die wasserlöslichen Typen liegen nach beendeter Kupplung als wäßrige Lösung vor, die entweder als solche weiterverwendet oder durch Einengen bzw. Fällungsreaktionen (z.B. als Thiuroniumsalze) aufgearbeitet werden kann.

Die Azoverbindungen der vorliegenden Erfindungen sind technisch außerordentlich interessante Stoffe mit mannigfaltigen Verwendungsmöglichkeiten.

Wegen ihrer Lichtempfindlichkeit eignen sie sich z.B. für den Einsatz in der Reproduktionstechnik, indem man sie zusammen mit geeigneten Kupplungskomponenten in Papiere oder Folien einarbeitet.

Bei der Einwirkung von UV- oder Sonnenlicht werden die erfindungsgemäßen Azoverbindungen gespalten, wobei die Spaltprodukte in Gegenwart der zugesetzten Kupplungskomponente einen anderen Farbstoff bildet. Zweckmäßigerweise wird diese Kupplungskomponente so gewählt, daß ein von der Ursprungsfarbe abweichender Farbton entsteht.

Geeignete Kupplungskomponenten sind solche, die bei einem $pH < 7$ rasch kuppeln, wie z.B.:
1-Hydroxy-8-amino-3,6-naphthalindisulfonsäure,
1-Hydroxy-8-acetylamino-3,6-naphthalindisulfonsäure,
1-Hydroxy-8-amino-3,5-naphthalindisulfonsäure,
1-Hydroxy-6-amino-3-naphthalinsulfonsäure,
1-Hydroxy-6-amino-4,8-naphthalindisulfonsäure,
1-Amino-6-hydroxy-3,8-naphthalindisulfonsäure,
1,8-Diamino-3,6-naphthalindisulfonsäure,
1,8-Diaminonaphthalin-4-sulfonsäure,
1-Amino-6-naphthalinsulfonsäure,
1-Hydroxy-7-amino-3-naphthalin-sulfonsäure,

1-Amino-3-naphthalinsulfonsäure,
1-Amino-3,6-naphthalindisulfonsäure,
1-Amino-3,8-naphthalindisulfonsäure,
1-Amino-3,6,8-naphthalintrisulfonsäure,
2-Amino-4,8-naphthalindisulfonsäure,
1-Hydroxy-3-naphthalinsulfonsäure,
1-Hydroxy-3,6-naphthalindisulfonsäure,
1-Hydroxy-3,8-naphthalundisulfonsäure,
2-Hydroxy-3,6-naphthalindisulfonsäure,
2-Hydroxy-3,7-naphthalindisulfonsäure.

Als Trägermaterialien kommen praktisch alle auch für das Diazotypieverfahren geeigneten Materialien, insbesondere Cellulosepapier und Celluloid, in Betracht.

Darüber hinaus können Verbindungen der Formel (I) als Schädlingsbekämpfungsmittel, insbesondere als Insektizide, Akarizide und Nematizide, eingesetzt werden. Besondere Wirkung zeigen diese Stoffe gegenüber verschiedenen Fliegen-, Käferlarven-, Raupen-, Mücken-, Blattlaus- und Spinnmilbenarten.

Weiterhin stellen die neuen Azoverbindungen wertvolle Ausgangsmaterialien zur Herstellung von Pyrazolonen dar, die ihrerseits Verwendung als Kupplungskomponenten zur Herstellung von Azofarbstoffen und als photographische Entwickler finden.

Die Pyrazolonsynthese ist dadurch gekennzeichnet, daß man man die Verbindungen der Formel (I) mit organischen oder anorganischen Basen behandelt.

Als Basen kommen zunächst die weiter oben genannten säurebindenden Mittel in Betracht. Weiter können folgende Lewis-Basen eingesetzt werden:

Alkali- oder Erdalkali- arylsulfinate, -perchlorate, -chloride, -fluoride, -nitrite, -thiocyanate, -thiosulfate, -alkoholate, -phenolate.

Die Reaktion wird in wäßrigen, wäßrigorganischen oder rein organischen Medien bei Temperaturen von -10°C bis 100°C, vorzugsweise bei 20-80°C, durchgeführt.

Zur Isolierung der Reaktionsprodukte wird das Reaktionsgemisch durch Zugabe von Säuren neutral oder sauer gestellt, wobei die Pyrazolone sich in fester oder öliger Form abscheiden. Die Reingewinnung erfolgt in üblicher Weise.

Bei dieser Synthese fallen stets Mischungen von Pyrazolonen an, die den Formeln

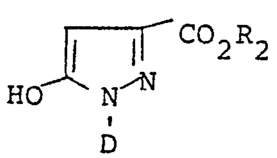

(Va)

und

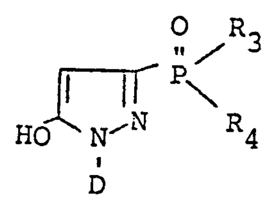

(Vb)

entsprechen,

worin die Reste die obengenannte Bedeutung haben.

Diese Gemische können jedoch gewünschtenfalls durch Chromatographie an z.B. Kieselgel-Säulen mit geeigneten Lösungsmittelgemischen, wie Essigester/ Methanol, getrennt werden. Das Mischungsverhältnis der entstehenden Pyrazolone kann durch Wahl der Lewis-Basen beeinflußt werden. Beispielsweise erfolgt bei nichthydrophilierten Azophosphonestern (vgl. S. 6) durch Zusatz von niederen Dialkylaminen eine Verschiebung zu Gunsten des Typs (Va). Bei sulfogruppenhaltigen, d.h. hydrophilierten Azophosphonestern gelingt diese Verschiebung durch Zusatz von Alkali- oder Erdalkalifluoriden. Dabei können allerdings toxische, durch Alkali hydrolysierbare, organische Fluorphosphorverbindungen als Nebenprodukte entstehen (Zur Struktur und Toxizität dieser Nebenprodukte vgl.: J. Chem. Soc. (London) 695 (1948)).

Durch Verseifung der Carbonsäureestergruppen können Pyrazoloncarbonsäuren hergestellt werden, die als Kupplungskomponenten zur Herstellung von Azofarbstoffen technische Bedeutung besitzen.

Vorteilhafterweise werden Pyrazolonsynthese und Verseifung als "Eintopfverfahren" durchgeführt.

Beispiel 1

Eine Lösung von 37 g Phosphonobernsteinsäuretretamethylester in 100 ml Wasser werden bei 0-5°C mit 150 ml wäßriger Lösung des Diazoniumsalzes aus 13,5 g Anilin versetzt. Durch Zugabe von 20 %iger Sodalösung bei 5-10°C und pH 8,0-8,5 wird gekuppelt. Nach beendeter Reaktion (kein pH-Abfall zu beobachten) wird die Mischung mit Essigester ausgeschüttelt. Die organische Phase wird über Magnesiumsulfat getrocknet und im Vakuum abgezogen. Man erhält 51,4 g der Verbindung

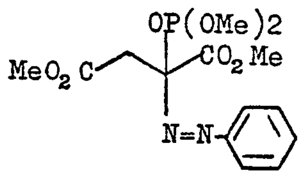

in Form eines gelb-braunen Öls, das nicht unzersetzt destillierbar ist.

$^1$H-NMR-Spektrum($CDCl_3$/TMS)

$\delta$ = 3.5 ppm Doublett (zentriert) 2-Methylenprotonen

$\delta$ = 3.6 - 4,1 ppm 4 Singuletts 12 Methylesterprotonen

$\delta$ = 7.3 - 8,0 ppm Multiplett 5 aromat. Protonen

Beispiel 2

Eine Lösung von 40,5 g Phosphonobernsteinsäuretetramethylester in 40 ml Wasser werden bei 0-5°C zu einer Diazoniumsalzlösung aus 28,9 g 4-Aminoazobenzol in 600 ml Wasser gegeben. Um das bei der Kupplung entstehende Produkt weitgehend in Lösung zu halten (bessere Rührbarkeit der Mischung) werden 200 ml MeOH zugegeben. Durch Zugabe von 20 %iger Sodalösung wird bei 5-10°C und bei pH 7,5-8,5 gekuppelt. Nach beendeter Reaktion (kein pH-Abfall) wird die Lösung mit Methylenchlorid ausgeschüttelt. Die organische Phase wird über Magnesiumsulfat getrocknet und im Vakuum abgezogen. Man erhält 67 g der Verbindung:

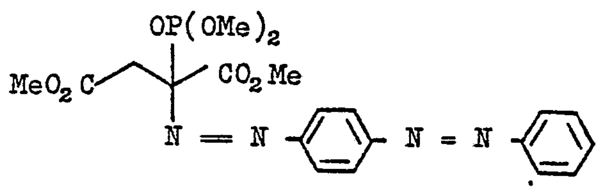

die zunächst als rotbraunes Öl anfällt, beim Stehenlassen jedoch kristallisiert. Fp 100-102°C aus Essigester.

$^1$H-NMR-Spektrum (DMSO-$d_6$/TMS)

$\delta$ = 3.5 ppm Multiplett (zentriert) $J_{12}$=7Hz 2 Methylenprotonen

$\delta$ = 3.6 - 4.1 ppm 3 Singuletts 12 Methylesterprotonen

$\delta$ = 7.5 - 8.3 ppm Multiplett 9 aromat. Protonen

Analog zu Beispiel 1 und 2 sind folgende Verbindungen darstellbar:

| Bsp. | Verbindung | $^1$H-NMR-Spektrum (Lösungsmittel/Standard) | Sonstige Physikal. Daten |
|---|---|---|---|
| 3 | 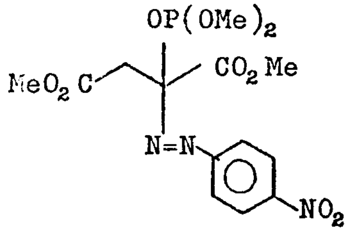 | $CDCl_3$/TMS<br>$\delta$ = 3,55 ppm Doublett, $J_{12}$=6Hz 2 Methylenprot.<br>$\delta$ = 3,6-4,5 ppm 4 Singuletts I2 Methylesterprot.<br>$\delta$ = 7.6-8.55 ppm AA'BB'-System 4 aromat.Prot. | Fp.: 86-89°C |
| 4 | 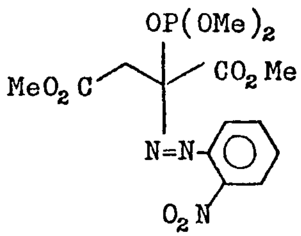 | $CDCl_3$/TMS<br>$\delta$ = 3.5 ppm Doublett, $J_{12}$=6Hz 2 Methylenprot.<br>$\delta$ = 3.6-4.2 ppm 4 Singuletts 12 Methylesterprot.<br>$\delta$ = 7.65-8.55 ppm AA'BB'-System 4 aromat.Prot. | Fp.: 80-83°C |
| 5 | 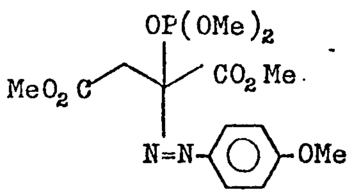 | $CDCl_3$/TMS<br>$\delta$ = 3.4 ppm Doublett, $J_{12}$=7Hz 2 Methylenprot.<br>$\delta$ = 3.55-4,0 ppm 4 Singuletts 12 Methylprot.<br>$\delta$ = 6.8-7.9 ppm AA'BB'-System 4 aromat.Prot. | Öl, $R_f$-Wert ($CH_3CO_2Et$/MeOH=8:2): 0.74 |

| Bsp. | Verbindung | ¹H-NMR-Spektrum | Sonstige Physikal.Daten |
|---|---|---|---|
| 6 | 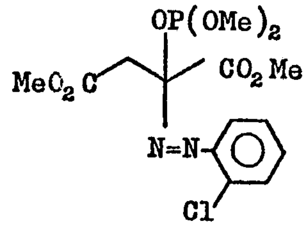 | $CDCl_3$/TMS<br>δ = 3,5 ppm Doublett, $J_{12}$=6Hz 2 Methylenprot.<br>δ = 3,6-4,2 ppm 4 Singulets I2 Methylesterprot.<br>δ = 7.1-7.8 ppm Multiplett 4 aromat. Prot. | Fp.: 74-77°C |
| 7 | 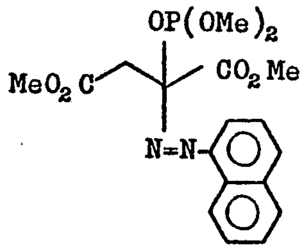 | $CDCl_3$/TMS<br>δ = 3.55 ppm Doublett $J_{12}$=6Hz 2 Methylenprot.<br>δ = 3.6-4.2 ppm 5 Singuletts, 12 Methylprot.<br>δ = 7.2-8.8 ppm Multiplett, 7 arom.Prot. | Fp.: 101-102°C<br>Analyse: C H N O P<br>ber.: 52.9 5.2 6.9 27.5 7.6<br>gef.: 53.0 5.2 7.0 26.8 8.0 |
| 8 | 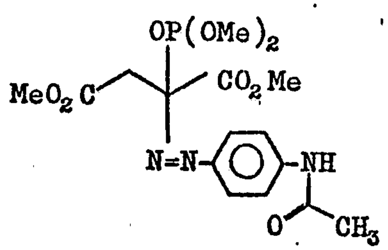 | $CDCl_3$/TMS<br>δ = 2.2 ppm Singulett 3 Acetylprot.<br>δ = 3.42 ppm Doublett, $J_{12}$=7Hz 2 Methylenprot.<br>δ = 3.6-4.2 ppm 6 Singuletts I2 Methylesterprot.<br>δ = 7.5-7.9 ppm Singulett(breit) 4 aromat.Prot.<br>δ = 9.3-9.6 Singulett 1 NH-Prot. | Fp.: 161-163°C |

| Bsp. | Verbindung | $^1$H-NMR-Spektrum | sonstige physikal. Daten |
|---|---|---|---|
| 9 | 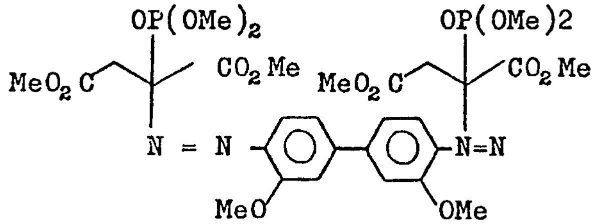 | $CDCl_3$/TMS<br>$\delta$ = 3.45 ppm Doublett, $J_{12}$=6Hz 2 Methylenprot.<br>$\delta$ = 3.6-4.2 ppm 6 Singuletts 30 Methylprot.<br>$\delta$ = 7.1-7.8 ppm Multiplett 6 aromat. Prot. | $R_f$-Wert/$EtO\overset{O}{\overset{\|}{C}}CH_3$ 0.30 |
| 10 | 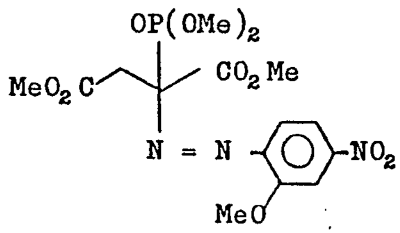 | $\delta$ = 3.45 ppm Doublett, $J_{12}$=6Hz 2 Methylenprot.<br>$\delta$ = 3.5-4.2 ppm 6 Singuletts 15 Methylprot.<br>$\delta$ = 7.3-8.0 ppm Multiplett 3 aromat. Prot. | Fp.: 125-127°C |
| 11 | 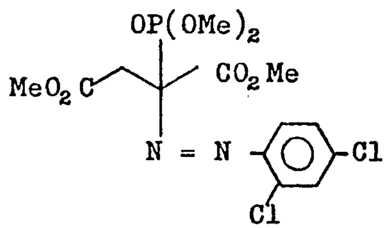 | $\delta$ = 3.47 ppm Doublett, $J_{12}$=6Hz 2 Methylenprot.<br>$\delta$ = 3.55-4.2 ppm 5 Singuletts 12 Methylprot.<br>$\delta$ = 7.1-7.8 ppm Multiplett 3 aromat. Prot. | Fp.: 124-127°C |

In Analogie zu den Beispielen 1-11 können folgende Azophosphonester dargestellt werden, die alle durch die charakteristischen $^1$H-NMR-Absorptionen bei $\delta$ = 3.4-3.5 ppm, Doublett für die beiden Methylenprotonen in $\beta$-Stellung zur Azo- und Phosphono-Gruppierung gekennzeichnet sind ($CDCl_3$ als Lösungsm. , TMS als Standard):

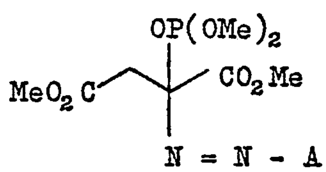

A =

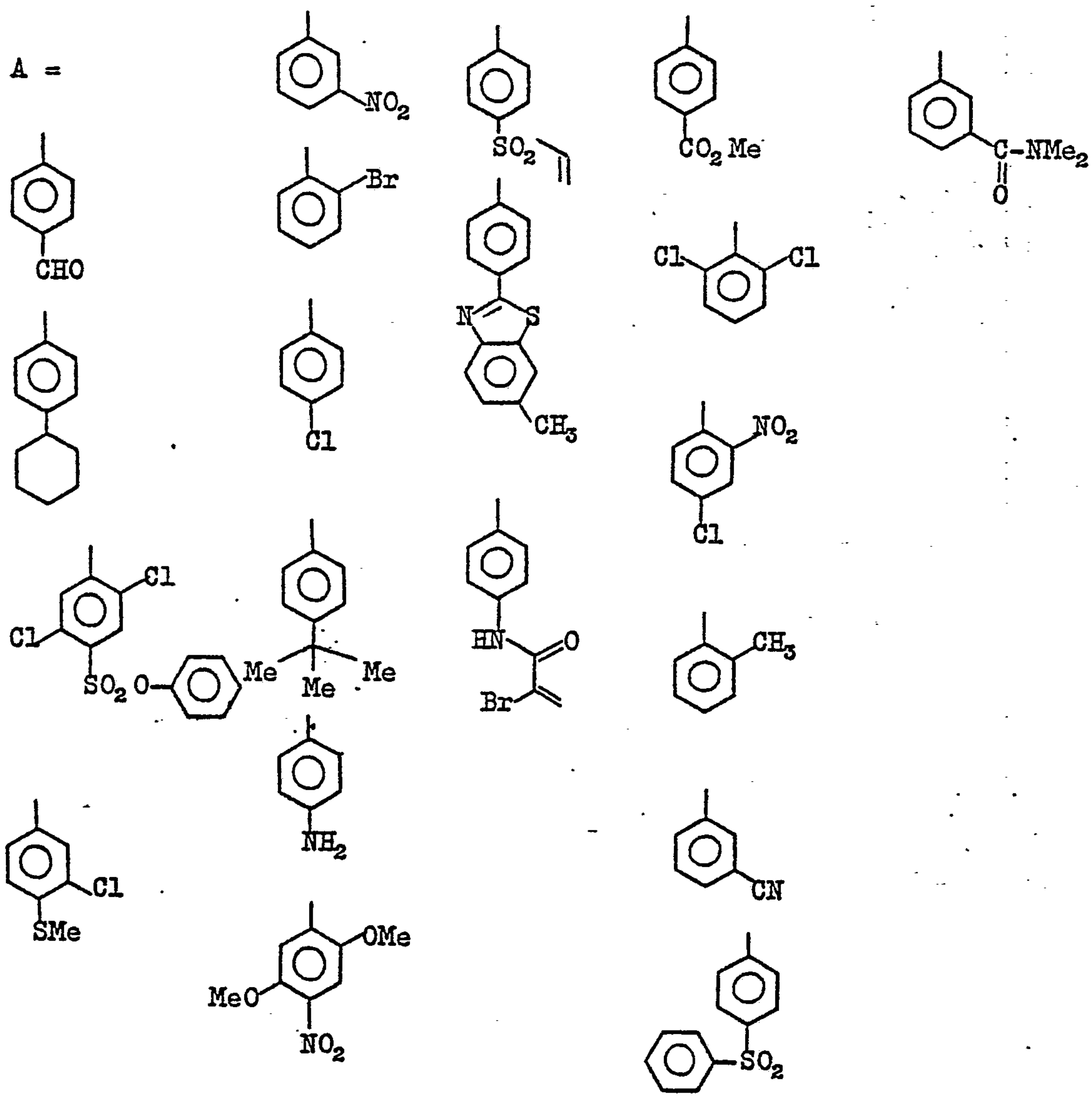

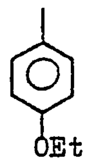

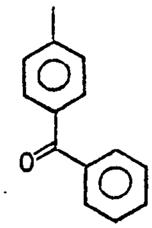

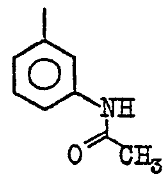

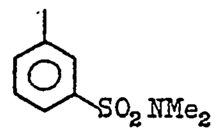

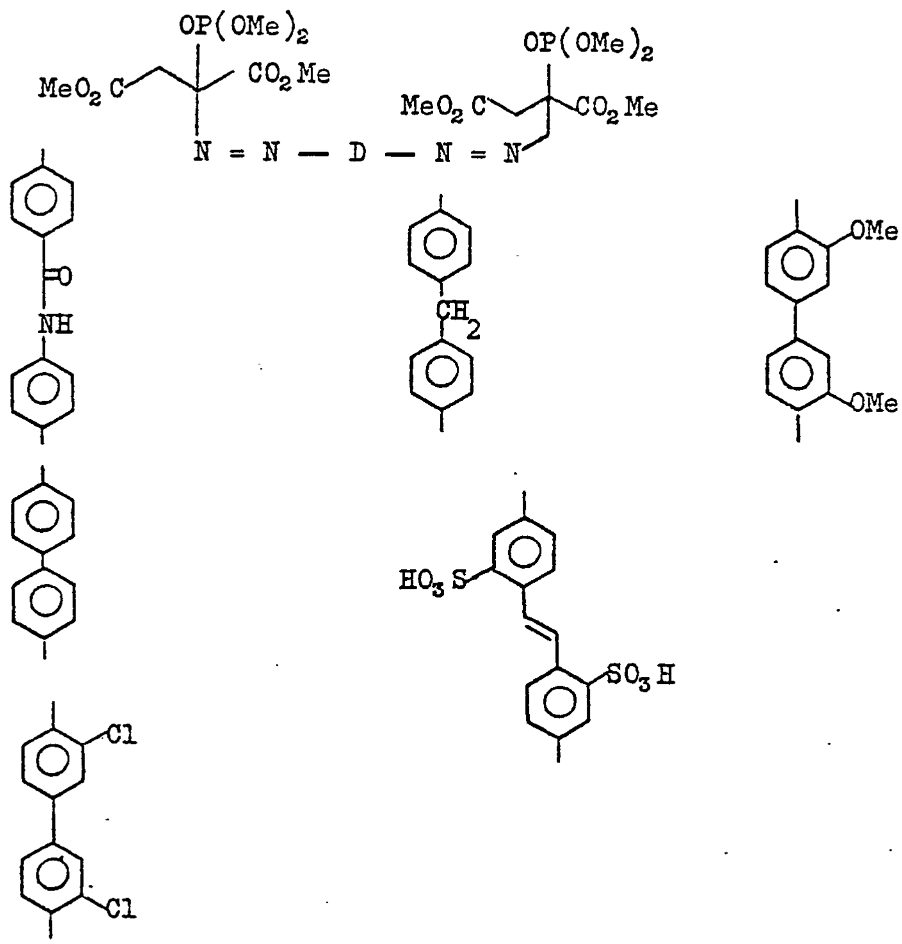

D =

Beispiel 12

Zu einer Diazoniumsalzlösung aus 173 g p-Sulfanilsäure (100 %ige Ware) in 1 Liter Wasser werden bei 0°C 282 g Phosphonobernsteinsäuretetramethylester gegeben. Bei 5-10°C wird bei pH 7,5-8,0 durch langsame Zugabe von 135 ml 40 %iger Natonlauge gekuppelt. Die Kupplung ist beendet, wenn kein pH-Abfall mehr zu beobachten ist.

Der entstandene Azophosphonobernsteinsäuretetramethylester ist durch seinen $R_f$-Wert von 0,7 1 auf Papier, Fließmittel: Butanol: Eisessig:Wasser = 80:20:40 charakterisiert. Er wird durch Besprühen des Chromatogramms mit einer Lösung aus N-Acetyl-H-Säure und anschließendes Belichten des feuchten Papiers sichtbar gemacht (rote Färbung).
Der Azophosphonester kann durch Fällen mit Benzyl-isothiuroniumchlorid aus essigsaurer Lösung als gelb-braunes, erstarrtes Öl isoliert werden:

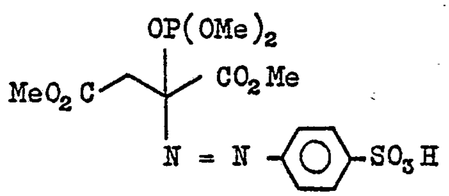

·

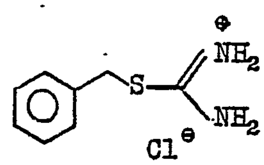

Das $^1$H-NMR-Spektrum dieses Salzes zeigt neben den Signalen für die Thiuroniumstruktur die auch bei den nicht-hydrophilierten Azophosphonester beobachteten, eindeutigen Signalgruppierungen.

| | | |
|---|---|---|
| $\delta$ = 3.3 ppm | Doublett, $J_{12}$ = 6Hz | 2 Methylenprotonen |
| $\delta$ = 3.4-3.9 ppm | 3 Singuletts | 12 Methylesterprotonen |
| $\delta$ = 7.4-8.0 ppm | AA'BB'-System | 4 aromatische Protonen |

Beispiel 13:

Zu einer Diazoniumsalzlösung aus 344 g 6-Aminotoluol-3-sulfonsäure (54,2 %ige Ware) in 1030 ml Wasser wird bei 0-5°C 294 g Phosphonobernsteinsäuretetramethylester gegeben. Es wird durch Zugabe von insgesamt 135 ml 40 %iger Natronlauge bei pH 8.5 während 1 Stunde gekuppelt. Dabei wird durch Zugabe von 300 g Eis die Temperatur bei 5-10°C gehalten. Das Ende der Kupplung wird daran erkannt, daß kein pH-Abfall mehr zu beobachten ist.

Der so dargestellte Azophosphonester ist durch seinen $R_f$-Wert von 0.72 auf Papier, Fließmittel: Butanol:Eisessig:Wasser = 80:20:40 charakterisiert. Er wird durch Besprühen des Chromatogramms mit einer Lösung aus N-Acetyl-H-Säure und anschließendes Belichten des feuchten Papiers sichtbar gemacht (rote Färbung).

Der Azophosphonester kann durch Fällen mit Benzylisothiuroniumchlorid aus essigsaurer Lösung als gelber Feststoff isoliert werden:

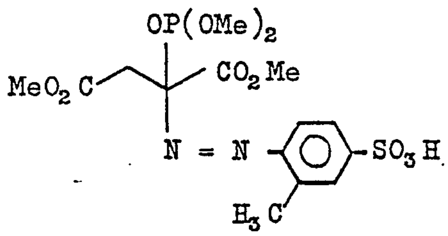

•

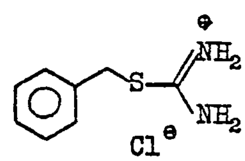

Das $^{1}$H-NMR-Spektrum dieses Salzes zeigt neben den Signalen für den Thiuroniumteil die auch bei den nicht-hydrophilierten Azophosphonestern beobachteten, typischen Signalgruppierungen: (DMSO-$d_6$ / TMS)

| | | | |
|---|---|---|---|
| $\delta$ = 2.47ppm | Singulett | 3 | Methylprotonen |
| $\delta$ = 3.40ppm | Doublett, $J_{12}$=6Hz | 2 | Methylenprotonen |
| $\delta$ = 3.50-4.05 ppm | 4 Singuletts | 12 | Methylesterprotonen |
| $\delta$ = 7.12-7.8 ppm | Multiplett | 8 | aromat. Protonen |

In Analogie zu den Beispielen 12 - 13 können folgende Azophosphonester dargestellt werden, die in ihren Benzylisothiuroniumsalzen alle durch die charakteristischen $^1$H-NMR-Signale bei $\delta$ = 3.3-3.4 ppm, Doublett für die beiden Methylenprotonen in $\beta$-Stellung zur Azo- und Phosphono-Gruppierung gekennzeichnet sind:

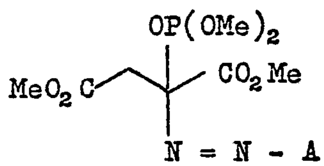

A =

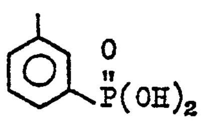

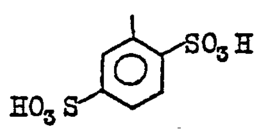

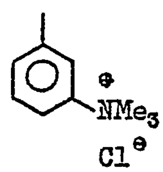

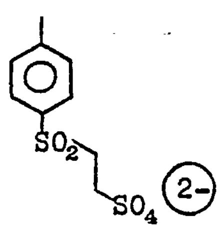

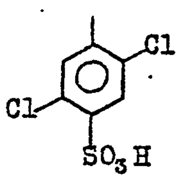

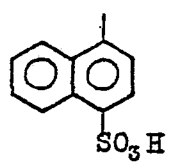

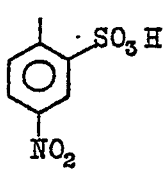

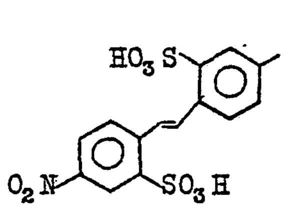

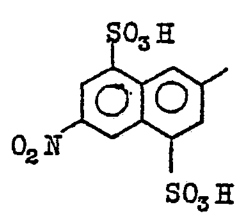

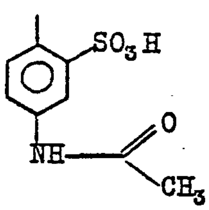

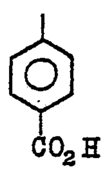

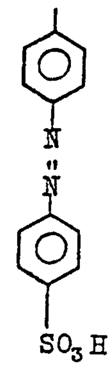

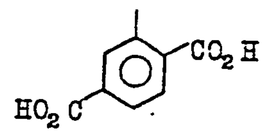

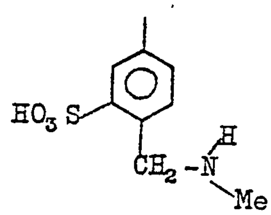

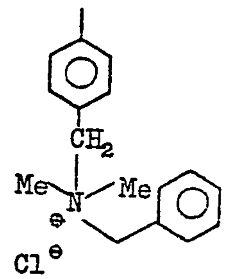

Beispiel 14

Cellulosepapier wird mit einer 0,3 molaren Lösung 4-Sulfophenylazophosphonobernsteinsäuretetramethylester (Darst.: Bsp. 12 ) und der N-Acetyl-H-Säure getränkt, 30 Minuten bei 50°C und 100 mm Hg Vakuum getrocknet und unter einer Vorlage belichtet. Das Muster der Vorlage erscheint in ca. 30 sec. als kräftiges, rotes Negativmuster auf leicht gelblichem Hintergrund. Das Abbild kann durch starkes Trocknen bei 160°C unter Lichtausschluß (Trockenschrank) gegenüber weiterer Lichteinwirkung fixiert werden.

Wie durch vergleichende DC-Chromatographie aus einer belichteten Lösung, die die oben genannten beiden Komponenten enthielt, gezeigt werden konnte, handelt es sich bei dem roten Farbstoff um das Kupplungsprodukt von diazotierter p-Sulfanilsäure auf N-Acetyl-H-Säure:

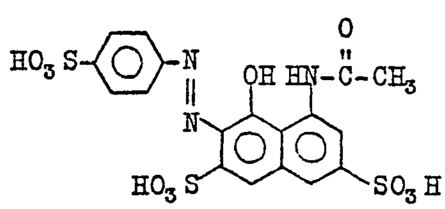

Beispiel 15

Bei gleicher Arbeitsweise wie im vorhergehenden Beispiel, jedoch unter Verwendung von 2-Methyl-4-Sulfophenylazophosphonobernsteinsäuretetramethylester (Beispiel 13) werden blaustichig rote Negativkopien mit gleichem Eigenschaftsbild erhalten.

Beispiel 16

Bei gleicher Arbeitsweise wie in Beispiel 14 , jedoch unter Verwendung des folgenden Bis-Azo-phosphonesters

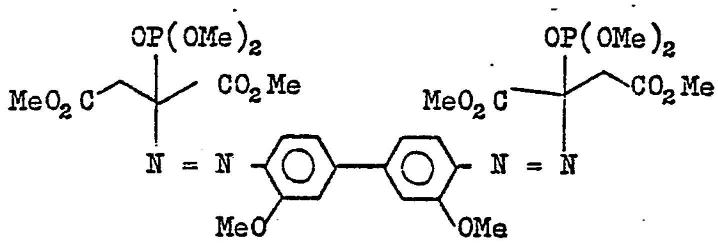

wird bei entsprechender Belichtung ein blau-violettes Farbmuster der Vorlage erhalten.

Beispiel 17

Eine durchsichtige Celluloid-Folie wird einseitig mit einer Gelatine-Schicht versehen. Diese Schicht wird mit einer Lösung aus:

40,6 g 4-Sulfophenylazophosphonobernsteinsäuretetramethylester (Beispiel 12),

36,1 g K-Säure-Natrium-Salz und

250 ml Wasser

getränkt. Die so präparierte und anschließend getrocknete Folie wird 5 Minuten in ein Wasserbad gelegt und unter einer Vorlage belichtet. Es entstehen scharfrandige, rote Abbildungsmuster der Vorlage in Form eines Negativbildes.

Beispiel 18

Cellulosepapier wird mit einer 0,3 molaren Lösung des 4-Sulfophenyl-phosphonobernsteinsäuretetramethylester (Darst. Bsp.: 12) getränkt, 30 Minuten bei 50°C und 100 mm Hg Vakuum getrocknet und unter einer Vorlage belichtet. Beim Besprühen oder Tränken des so vorbehandelten Papiers mit einer 0,3 molaren Lösung von N-Acetyl-H-Säure entsteht spontan ein rotes Negativ-Abbild der Vorlage in rotem Farbton, das durch scharfes Trocknen bei 160°C fixiert werden kann.

Beispiel 19

Die insektizide Wirkung der neuen Azoverbindungen geht beispielsweise aus folgender Tabelle hervor:

| Verbindung der Formel | Wirkung |
|---|---|
| 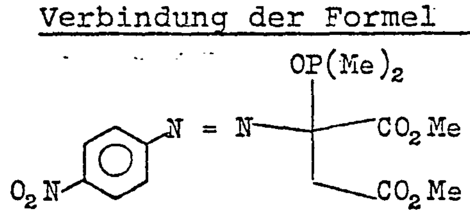 | 1) Taufliege (Drosophila melanogaster): 0,1 %ige Lösung: 100% Abtötung<br>2) Blattlaus (Doralis fabae), systemisch: 0,1%ige Lösung: 50% Abtötung |

| Verbindung der Formel | Wirkung |
|---|---|
| 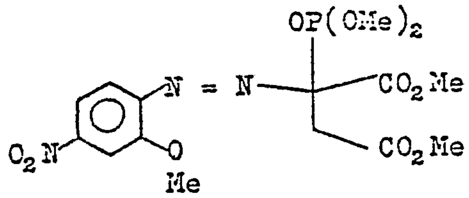 | Taufliege (Drosophila melanogaster): 0,01%ige Lösung: 90% Abtötung |
| 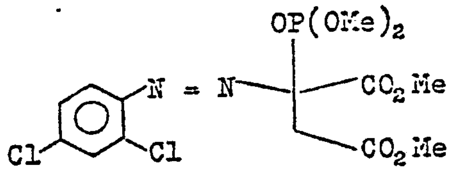 | Raupen (Plutella maculipennis): 0.1%ige Lösung: 100% Abtötung |

Beispiel 20

a) 3.58 g des gemäß Beispiel 1 hergestellten Phenylazophosphonesters werden in 50 ml gesättigter methanolischer Kaliumcarbonat-Lösung bei 0-5°C 1 Stunde gerührt. Es wird mit 30 %iger Salzsäure auf pH 3 angesäuert und mit Essigester mehrmals ausgeschüttelt. Die vereinigten organischen Phasen werden über Magnesiumsulfat getrocknet. Das Lösungsmittel wird unter Vakuum abgezogen. Man erhält 2,3 g eines Gemisches der Verbindungen

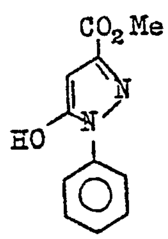

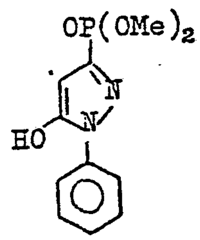

b) Zum reinen 1-Phenylpyrazolon-3-carbonester gelangt man, wenn 3,58 g des Phenylazophosphonesters in 50 ml Methanol bei 0-5°C mit 20 ml Diäthylamin versetzt und 1 Stunde gerührt werden. Bei analoger Aufarbeitung wie in Beispiel 20 a) erhält man 2,0 g des festen Carbomethoxypyrazolons, das durch chromatographischen Vergleich mit einer authentischen Probe identifiziert wurde.

Beispiel 21

9,26 g des gemäß Beispiel 2 hergestellten Azophosphonesters werden in 100 ml gesättigter methanolischer Kaliumcarbonat-Lösung 3 Stunden bei Raumtemperatur gerührt. Die Lösung wird mit 30 %iger Salzsäure auf pH 1 angesäuert und mehrmals mit Essigester extrahiert. Die vereinigten organischen Phasen werden über Magnesiumsulfat getrocknet und am Rotationsverdampfer abgezogen. Man erhält 6,0 g eines gelb-braunen Pulvers, das ein Gemisch der folgenden Verbindungen ist:

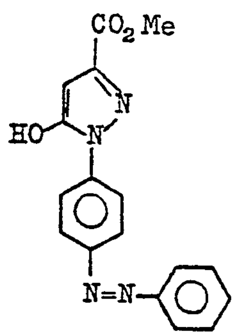

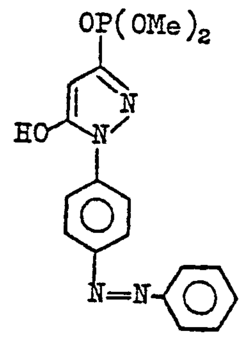

Beispiel 22

Die Lösung des 4-Sulfophenylazophosphonobernsteinsäuretetramethylesters gemäß Beispiel 12 wird mit 450 ml 40 %iger Natronlauge-Lösung versetzt und 3 Stunden bei 10-25°C gerührt. Die Vollständigkeit der Umsetzung wird chromatographisch aus einer angesäuerten Probe überprüft. Auf zwei getrennten Papierchromatogrammen werden aufgetragen: Azophosphonester-Lösung (Ausgangsverbindung), angesäuerte Reaktionslösung, authentische Probe des Carboxypyrazolons (A):

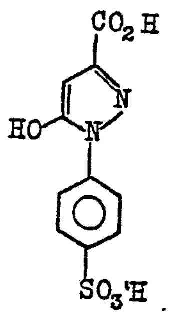

(A)

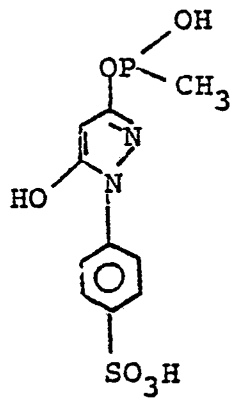

(B)

Es wird in Butanol : Eisessig : Wasser = 80 : 20 : 40 als Fließmittel auf Papier chromatographiert.

Zum Test auf Vollständigkeit des Ringschlußes wird danach eines der beiden Chromatogramme mit N-Acetyl-H-Säure besprüht und belichtet. Das Nicht-Auftreten einer roten Färbung zeigt an, daß der Azophosphonester vollständig umgesetzt ist. Das zweite Chromatogramm wird zum Nachweis der gebildeten Pyrazolone (A) und (B) mit diazotierter p-Nitroanilinlösung besprüht. Die Pyrazolone kuppeln mit intensiv gelber Farbe. $R_f$(A):0,30, $R_f$(B):0.16

Zur Isolierung des Carboxy-Pyrazolons (A) wird die Lösung mit 800 ml 30 %iger HCl so angesäuert, daß 40°C nicht überschritten werden. Es wird über Nacht nachgerührt. Durch Absaugen erhält man 270 g feuchtes salzhaltiges Pyrazolon.

Beispiel 23

Eine Lösung des gemäß Beispiel 13 hergestellten 6-Amino-toluol-3-sulfophenylazophosphonobernsteinsäuretetramethylesters wird mit 140 g fester Natriumhydroxid -Plätzchen versetzt. Die Lösung wird 16 Stunden nachgerührt. Es wird mit 350 ml 30 %iger HCl-Lösung versetzt und das Pyrazolon mit 300 g Kaliumchlorid ausgesalzen. Es wird über Nacht nachgerührt. Man erhält 735 g feuchtes, salzhaltiges Pyrazolon der Formel

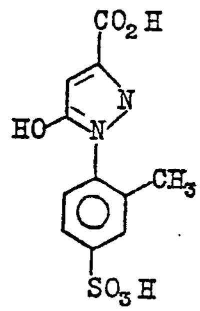

Beispiel 24

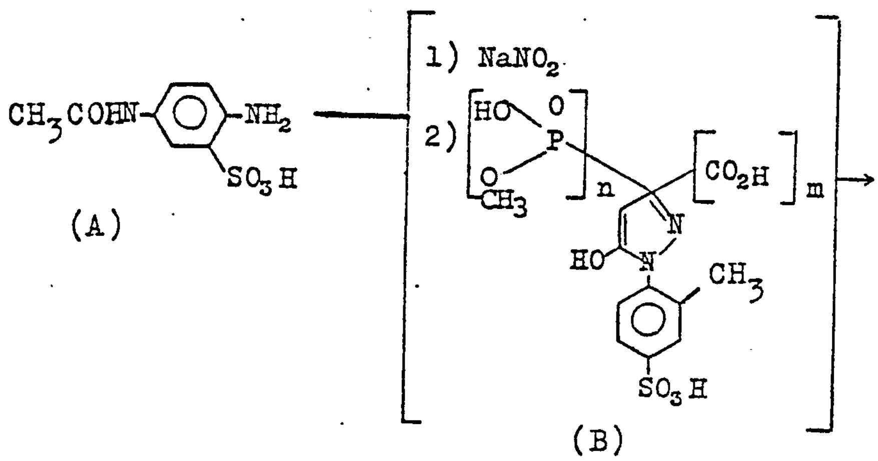


(A)

(B)

(C)

(m=1,wenn n=o)
(m=o,wenn n=1)

53.3g diazotierter Acetmetaminsäure (A) in 650 ml Wasser werden bei 10°C vorgelegt. Es werden o.223 mol des in Beispiel 13 dargestellten Gemisches von

Carboxy- und Phosphonopyrazolon (B) in 1/2 Stunde hinzugegeben. Es wird mit 80 ml 20 %iger NaOH auf pH 4 gestellt.

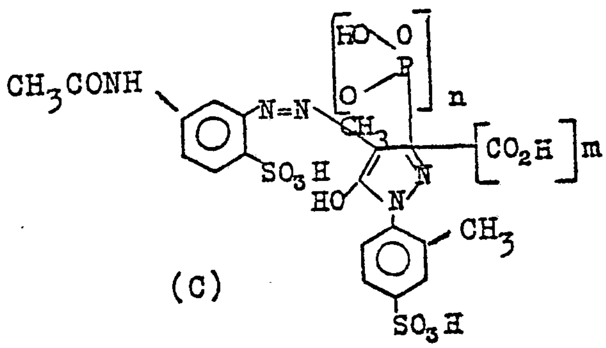

(C)

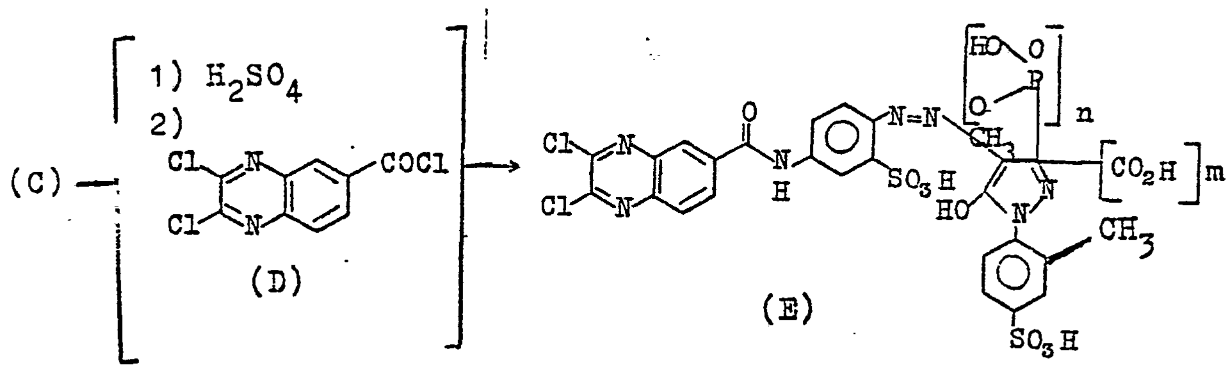


(D)

(E)

Zur Spaltung der N-Acetylgruppe wird die Azoverbindung (C) mit 750 ml 11 %iger Schwefelsäure versetzt und 1/2 Stunde auf 100°C erhitzt. Nach dem Abkühlen wird die erhaltene Suspension mit 350 ml 40 %iger NaOH neutral gestellt. Es werden 66.7 g des Dichlorchinoxalins (D) und 2 g eines Emulgators zugegeben. Bei 40°C wird pH 6 mit 20 %iger Sodalösung gehalten. Die neutralgestellte Farbstoff-Lösung wird zur Isolierung des Farbstoffgemischs (E) eingedampft bzw. sprühgetrocknet. Das Farbstoffgemisch (E) eignet sich hervorragend zum Färben von Baumwolle nach einem für Reaktivfarbstoffe üblichen Verfahren.

Patentansprüche

1. Azoverbindungen der Formel

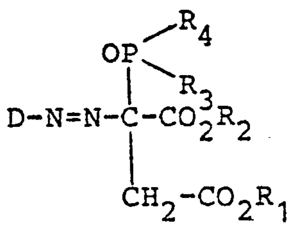

(I)

worin

D für den Rest einer Diazokomponente,

$R_1$ für gegebenenfalls substituiertes Alkyl, Aryl, Alkenyl, Cycloalkyl, Aralkyl oder Hetaralkyl,

$R_2$ für $R_1$,

$R_3$ für $OR_1$ oder 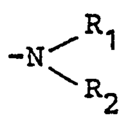 und

$R_4$ für $R_1$ oder $R_3$ stehen.

2. Azoverbindungen gemäß Anspruch 1, dadurch gekennzeichnet, daß

D für 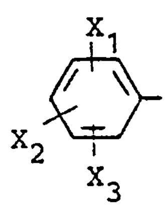

$R_1$ und $R_2$ für $C_1$-$C_4$-Alkyl, vorzugsweise $C_1$-$C_2$-Alkyl,

$R_3$ für $OR_1$ und

$R_4$ für $R_1$ oder - vorzugsweise - $OR_1$ stehen,

wobei $X_1$ Wasserstoff, Cl, Br, $CH_3$, $OCH_3$ oder $CF_3$

$X_2$ CN, $NO_2$ Phenylazo, $C_1$-$C_2$-Alkylcarbonylamino oder $CF_3$

$X_3$ CHO, $CO_2$-$C_1$-$C_2$-Alkyl, Phenylsulfonyl, $SO_3H$, $C_1$-$C_2$-Alkylcarbonyl, $C_1$-$C_2$-Alkylsulfonyl oder $X_2$ bedeuten.

3. Azoverbindungen gemäß Anspruch 1, dadurch gekennzeichnet, daß D für

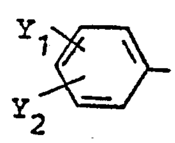

$R_1$ und $R_2$ für Methyl oder Ethyl und

$R_3$ und $R_4$ für $OR_1$ stehen, wobei

$Y_1$ Wasserstoff, Chlor oder Methyl und

$Y_2$ Nitro, $SO_3H$ oder $Y_1$ bedeuten.

4. Azoverbindung der Formel

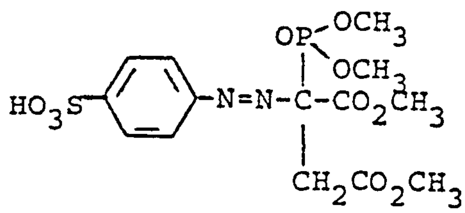

5. Azoverbindung der Formel

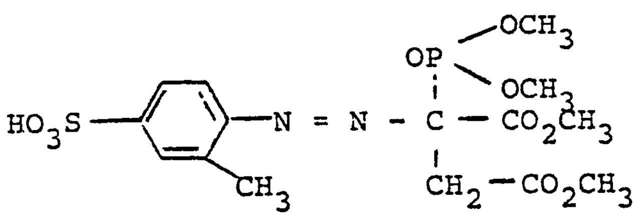

6. Azoverbindung der Formel

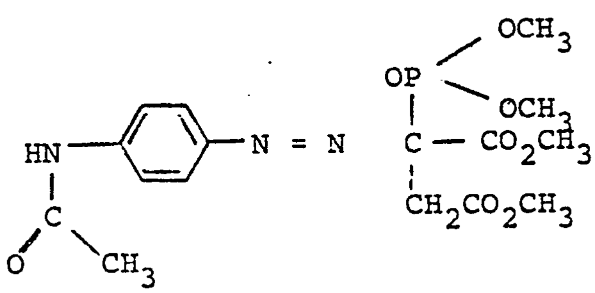

7. Azoverbindung der Formel

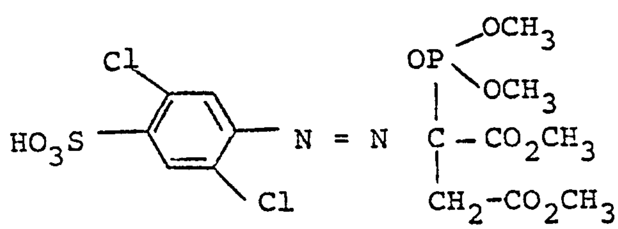

8. Verfahren zur Herstellung von Azoverbindungen, dadurch gekennzeichnet, daß man diazotierte Amine der Formel

$$D-NH_2$$

auf Phosphonobernsteinsäureester der Formel

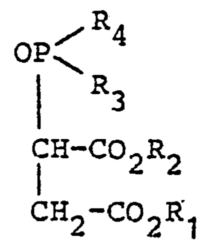

in Gegenwart säurebindender Mittel kuppelt.

9. Verwendung der Azoverbindungen gemäß Anspruch 1 als Insektizide oder als lichtempfindliche Materialien.

10. Verwendung der Azoverbindungen gemäß Anspruch 1 zur Synthese von Pyrazolonen durch Behandlung mit Basen.

0022512

Europäisches Patentamt

# EUROPÄISCHER RECHERCHENBERICHT

Nummer der Anmeldung

EP 80 10 3722

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | SOVIET INVENTIONS ILLUSTRATED, Section Chemicals Derwent Publications Ltd. Week W 34 (1975)<br>& SU - A - 451 706 (A.A. PETROV et al. )<br>* Zusammenfassung *<br>-- | 1 |
| | US - A - 2 087 706 (W.A. SEXTON)<br>* Ganze Dokument *<br>-- | 8 |
| DA | US - A - 3 801 682 (R.S. LUDINGTON)<br>* Ganze Dokument *<br>-- | 1 |
| DA | GB - A - 1 491 595 (ICI)<br>* Ganze Dokument *<br>---- | 1 |

**KLASSIFIKATION DER ANMELDUNG (Int Cl.³)**

C 07 F 9/40, 9/32, 9/44, 9/36, 9/65
C 09 B 29/32, 31/10, 35/035
G 03 C 1/52
C 07 D 231/22, 231/34
B 41 M 5/00

**RECHERCHIERTE SACHGEBIETE (Int. Cl.³)**

C 07 F 9/40, 9/32, 9/44, 9/36
C 09 B 29/32, 31/10, 35/035

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 25-09-1980 | BESLIER |